(19) Europäisches Patentamt
European Patent Office
Office européen des brevets

(11) Veröffentlichungsnummer: **0 074 023**
**A1**

(12) # EUROPÄISCHE PATENTANMELDUNG

(21) Anmeldenummer: 82107829.2

(22) Anmeldetag: 26.08.82

(51) Int. Cl.[3]: C 07 D 229/00
C 08 G 18/00

(30) Priorität: 08.09.81 DE 3135542

(43) Veröffentlichungstag der Anmeldung: 16.03.83 Patentblatt 83/11

(84) Benannte Vertragsstaaten: BE DE FR GB IT

(71) Anmelder: BAYER AG
Zentralbereich Patente, Marken und Lizenzen
D-5090 Leverkusen 1, Bayerwerk(DE)

(72) Erfinder: Kopp, Richard, Dr.
Wolfskaul 12
D-5000 Köln 80(DE)

(72) Erfinder: Grögler, Gerhard, Dr.
von-Diergardt-Strasse 46
D-5090 Leverkusen 1(DE)

(72) Erfinder: Rasshofer, Werner, Dr.
Wolfskaul 10
D-5000 Köln 80(DE)

(72) Erfinder: Hess, Heinrich, Dr.
Auf der Griesse 48
D-5090 Leverkusen 1(DE)

(54) **Uretdiongruppen aufweisendes Diisocyanat, ein Verfahren zur Herstellung von hitzevernetzbaren Oligoadditionsprodukten und deren Verwendung zur Herstellung von hochmolekularen Kunststoffen.**

(57) Die Erfindung betrifft das neue Uretdiongruppen aufweisende Diisocyanat der Formel

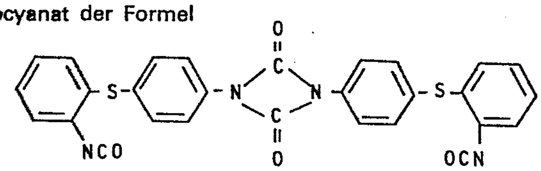,

ein Verfahren zur Herstellung von hitzevernetzbaren aromatisch gebundene primäre Aminogruppen und Uretdiongruppen aufweisenden Oligoadditionsprodukten durch Umsetzung des neuen Diisocyanats mit mindestens zwei aromatisch gebundene primäre Aminogruppen aufweisenden organischen Verbindungen des Molekulargewichtsbereichs 500 bis 10 000 unter Einhaltung eines $NCO/NH_2$-Molverhältnisses von 1:1,05 bis 1:2, sowie die Verwendung der bei diesem Verfahren erhältlichen Oligoadditionsprodukte zur Herstellung von Harnstoffgruppen aufweisenden hochmolekularen Kunststoffen durch Erhitzen der Oligoadditionsprodukte auf eine Temperatur von oberhalb 130°C.

EP 0 074 023 A1

Croydon Printing Company Ltd.

0074023

BAYER AKTIENGESELLSCHAFT 5090 Leverkusen, Bayerwerk
Zentralbereich
Patente, Marken und Lizenzen Wr/bo/c

Uretdiongruppen aufweisendes Diisocyanat, ein Verfahren zur Herstellung von hitzevernetzbaren Oligoadditionsprodukten und deren Verwendung zur Herstellung von hochmolekularen Kunststoffen

Die Erfindung betrifft ein neues Uretdiongruppen aufweisendes Diisocyanat, ein Verfahren zur Herstellung von hitzevernetzbaren, aromatisch gebundene primäre Aminogruppen und Uretdiongruppen aufweisenden Oligoadditionsprodukten unter Verwendung des Uretdiongruppen aufweisenden Diisocyanats, sowie die Verwendung der Oligoadditionsprodukte zur Herstellung von hochmolekularen Kunststoffen.

Uretdiongruppen aufweisende Diisocyanate bzw. Verfahren zu ihrer Herstellung durch Dimerisierung organischer, insbesondere aromatischer Diisocyanate sind bekannt (vgl. Kunststoff-Handbuch, Band VII, Polyurethane, herausgegeben von Vieweg-Höchtlen, Carl Hanser Verlag, München 1966). Die Herstellung derartiger Diisocyanate erfolgt im allgemeinen durch Zugabe eines Dimerisierungskatalysators wie z.B. Trialkylphosphinen (J. Org. Chem. 8, 23 (1943)), Aryl-alkyl-phosphinen (DE-OS

2 452 390), 3- bzw. 4-substituierten Pyridinen (GB-PS 821 158) zu einer Lösung des zu dimerisierenden Diisocyanats in einem geeigneten Lösungsmittel wie z.B. Toluol, Xylol oder Chlorbenzol. Bei Erreichen des gewünschten Dimerisierungsgrades wird die Reaktion durch Zugabe eines Katalysatorengifts abgebrochen.

Nunmehr wurde überraschenderweise gefunden, daß die Dimerisierung eines ganz speziellen Ausgangsdiisocyanats nämlich von 2,4'-Diisocyanato-diphenylsulfid zu dem nachstehend näher beschriebenen Uretdiongruppen aufweisenden erfindungsgemäßen Diisocyanat führt, das sich durch eine Reihe bemerkenswerter Vorteile auszeichnet.

Gegenstand der Erfindung ist das Uretdiongruppen aufweisende Diisocyanat der Formel

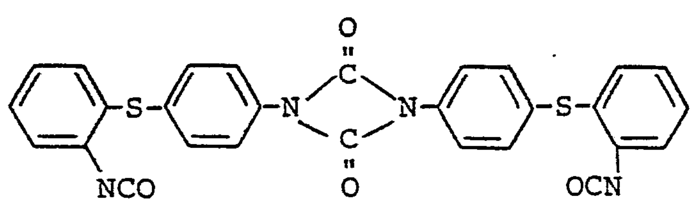

Gegenstand der Erfindung ist auch ein Verfahren zur Herstellung von hitzevernetzbaren, aromatisch gebundene primäre Aminogruppen und Uretdiongruppen aufweisenden Oligoadditionsprodukten, dadurch gekennzeichnet, daß man das Uretdiongruppen aufweisende Diisocyanat der Formel

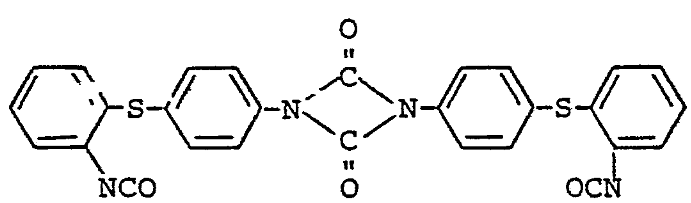

mit mindestens zwei aromatisch gebundene, primäre Aminogruppen aufweisenden organischen Verbindungen des Molekulargewichtsbereichs 500 bis 10 000 unter Einhaltung eines $NCO/NH_2$-Molverhältnisses von 1:1,05 bis 1:2 umsetzt.

Gegenstand der Erfindung ist auch die Verwendung der erfindungsgemäß erhältlichen, aromatisch gebundene primäre Aminogruppen und Uretdiongruppen aufweisenden Oligoadditionsprodukte zur Herstellung von Harnstoffgruppen aufweisenden, hochmolekularen Kunststoffen, dadurch gekennzeichnet, daß man die Oligoadditionsprodukte auf eine Temperatur von oberhalb 130°C erhitzt.

Das erfindungsgemäße, Uretdiongruppen aufweisende Diisocyanat wird in an sich bekannter Weise durch Dimerisierung von 2,4'-Diisocyanato-diphenylsulfid erhalten. Dieses als Ausgangsmaterial einzusetzende Diisocyanat kann beispielsweise gemäß der Lehre der DE-OS 2 916 135 (entsprechend US-Patentanmeldung 136 624 vom 02.04.1980) erhalten werden.

Die Dimerisierungsreaktion erfolgt nach den an sich bekannten Methoden des Standes der Technik. Beispielsweise kann man hierzu dergestalt vorgehen, daß man das genannte Ausgangsdiisocyanat als Lösung in einem inerten Lösungsmittel vorlegt und die Lösung mit einem geeigneten Dimerisierungskatalysator insbesondere mit einem Trialkylphosphin wie z.B. Triethylphosphin oder

Tributylphosphin bei 0 bis 80°C versetzt. Die Katalysatoren werden in üblichen Mengen z.B. von 0,05 bis 5 Gew.-%, vorzugsweise 0,1 bis 1,0 Gew.-% und besonders bevorzugt von 0,1 bis 0,35 Gew.-%, bezogen auf zu dimerisierendes Ausgangsdiisocyanat, eingesetzt. Als Lösungsmittel sind im Prinzip alle gegenüber Isocyanatgruppen inerte Lösungsmittel geeignet, bevorzugt werden jedoch unpolare bis schwach polare Lösungsmittel oder deren Gemische, wie z.B. Hexan, Octan, Cyclohexan, Petrolether, Toluol, Chlorbenzol, Essigsäureethylester, Aceton oder Diethylether, eingesetzt. Die Dimerisierung kann beim berechneten NCO-Gehalt der Lösung durch Zugabe eines Katalysatorengifts, beispielsweise eines Alkylierungsmittels wie z.B. Toluolsulfonsäuremethylester abgebrochen werden. Das dimere Diisocyanat ist in den verwendeten Lösungsmitteln im Unterschied zum Ausgangsdiisocyanat oftmals unlöslich und kann somit als spontan ausfallender Feststoff durch Filtrieren isoliert werden.

Reaktionspartner für das erfindungsgemäße Diisocyanat bei der Durchführung des erfindungsgemäßen Verfahrens sind mindestens zwei aromatisch gebundene, primäre Aminogruppen aufweisende, organische Verbindungen mit einem dampfdruckosmometrisch bestimmbaren mittleren Molekulargewicht von mindestens 500, die, von diesen Aminogruppen abgesehen, gegenüber Isocyanat- und Uretdiongruppen inert sind.

Vorzugsweise werden dieser Definition entsprechende aromatische Di- bzw. Polyamine eingesetzt, die bei

Raumtemperatur Flüssigkeiten oder die niedrigschmelzende bis wachsartige Verbindungen darstellen. Besonders bevorzugt werden endständig Aminoaryl-verschlüsselte, gegebenenfalls Urethangruppen aufweisende Polyether oder Polyester mit zwei endständigen, aromatisch gebundenen, primären Aminogruppen eines über 500, vorzugsweise zwischen 500 und 6000 liegenden, dampfdruckosmometrisch bestimmbaren Molekulargewichts eingesetzt. Derartige Verbindungen können nach an sich bekannten Verfahren, wie sie beispielsweise in DE-OS 2 948 419, DE-OS 2 546 536, US-PS 4 224 417, DE-AS 1 270 046, GB-PS 1 117 494, DE-AS 1 694 152, US-PS 3 625 871, FR-PS 1 415 317, US-PS 3 385 829 oder der deutschen Patentanmeldung P 31 31 252.7 beschrieben sind, erhalten werden.

Bei der Durchführung des erfindungsgemäßen Verfahrens kommen die Reaktionspartner in solchen Mengen zum Einsatz, daß im Reaktionsgemisch für jede Isocyanatgruppe des erfindungsgemäßen Uretdion-Diisocyanats 1,05 bis 2, vorzugsweise 1,4 bis 2 Aminogruppen des Polyamins zur Verfügung stehen. Vorzugsweise erfolgt die Umsetzung in Gegenwart eines organischen Lösungsmittels der bereits oben beispielhaft genannten Art. Besonders gut geeignet ist eine Arbeitsweise, gemäß welcher die Aminkomponente im organischen Lösungsmittel bei ca. 0 - 120°C, vorzugsweise 30 - 100°C vorgelegt wird und dann das erfindungsgemäße Diisocyanat in fein gemahlter Form eingetragen wird. Selbstverständlich kann auch umgekehrt verfahren werden, und zwar dergestalt, daß man das Diisocyanat in einem Lösungsmittel der genannten Art gelöst oder insbesondere suspendiert vorlegt und die Aminkomponente in dem gleichen oder einem anderen Lösungsmittel gelöst zugibt. Die Umsetzung ist im allgemeinen beendet, wenn im Reaktionsgemisch IR-spektrometrisch keine Isocyanatgruppen mehr nachweisbar sind. In der Praxis ist es allerdings nicht immer erforderlich, die Reaktion bis zum völligen Verschwinden der Isocyanatgruppen zu führen. Die Isolierung der Umsetzungsprodukte kann im Falle der Verwendung von apolaren oder wenig polaren Lösungsmitteln, in denen die Umsetzungsprodukte nicht löslich sind, durch Filtration geschehen. Im Falle der Verwendung von polaren Lösungsmitteln, in denen die Umsetzungsprodukte löslich sind, erfolgt deren Isolierung beispielsweise durch destillative Entfernen des Lösungsmittels im Vakuum oder auch unter Normaldruck, wobei jedoch darauf geachtet werden muß, daß die Temperatur 100°C, vorzugsweise 50°C nicht übersteigt. Die erfindungsgemäßen Verfahrensprodukte fallen dann als Rückstand an.

Es ist nun besonders bemerkenswert, daß die erfindungsgemäßen Verfahrensprodukte im allgemeinen einen Schmelzpunkt im Bereich von ca. 130 - 150°C aufweisen und bereits bei einer Temperatur, die geringfügig oberhalb dieses Schmelzpunktes liegt, vorzugsweise im Bereich von 140 - 180°C vernetzt werden können. Diese Selbstvernetzung ist auf eine Reaktion der freien Aminogruppen mit den Uretdionringen unter Ausbildung von Biuret-und/oder Harnstoffgruppen zurückzuführen. Dieser Vernetzungsmechanismus ist einerseits daran erkennbar, daß die aufgeschmolzenen, erfindungsgemäßen Verfahrensprodukte nach kurzer Zeit Fäden ziehen und andererseits daran, daß beim Erhitzen der erfindungsgemäßen Verfahrensprodukte auf die genannten Temperaturen die für die Uretdionringe charakteristische Carbonyl-Bande bei 1780 $cm^{-1}$ und die für den Ring charakteristische Bande bei 1340 $cm^{-1}$ im IR-Spektrum alsbald verschwinden.

Es ist überraschend, daß die erfindungsgemäßen Verfahrensprodukte trotz des Vorliegens von Harnstoffgruppen bei relativ niedrigen Temperaturen schmelzen und bereits bei diesen relativ niedrigen Temperaturen selbstvernetzend sind. Die erfindungsgemäßen Verfahrensprodukte stellen somit wertvolle Ausgangsmaterialien zur Herstellung von hochmolekularen Kunststoffen dar. Diese erfindungsgemäße Verwendung der erfindungsgemäßen Verfahrensprodukte geschieht durch einfaches Erhitzen der erfindungsgemäßen Verfahrensprodukte auf oberhalb 130°C, vorzugsweise auf 140 - 180°C. Den erfindungsgemäßen Verfahrensprodukten können selbstverständlich vor dieser Endvernetzung beliebige Hilfs- und Zusatzmittel wie z.B. Füllstoffe, Pigmente oder Alterungsschutzmittel einverleibt werden. Die Hitzevernetzung kann im Anschluß oder gleichzeitig mit einer Formgebung erfolgen, gegebenenfalls unter äußerem Druck.

Die nachstehenden Beispiele dienen zur weiteren Erläuterung der Erfindung. Alle Prozentangabe beziehen sich, soweit nicht anderslautend vermerkt, auf Gewichtsprozente.

Beispiel 1

Herstellung des erfindungsgemäßen, Uretdiongruppen aufweisenden Diisocyanats

Zu einer Lösung von 1250 g destilliertem 2,4'-Diisocyanto-diphenylsulfid in einem Gemisch aus 1250 g Toluol und 1250 g Petrolether werden bei 20°C 6,25 g Tributylphosphin zugesetzt. Die sich allmählich bildende Suspension wird während 2,5 Stunden bei ca. 20°C intensiv gerührt. Schließlich wird die Reaktion durch Einrühren von 6,25 g Toluolsulfonsäuremethylester abgebrochen. Nach weiterem 10-minütigem Rühren wird der Festkörper abfiltriert und mit Ether gewaschen. Anschließend wird bei Raumtemperatur unter Vakuum getrocknet.

Es wurden auf diese Weise 1100 g (88 % der Theorie) des Diisocyanats der Formel

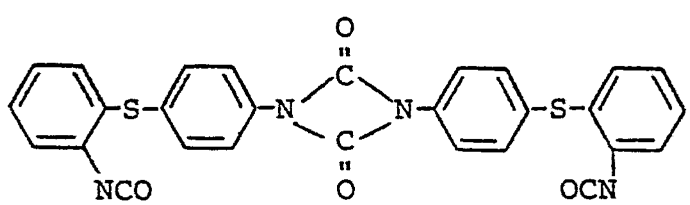

erhalten. Das Diisocyanat weist einen Schmelzpunkt von 178 - 180°C und einen NCO-Gehalt von 15,9 % (berechnet: 15,67 %) auf.

Die Struktur wurde durch $^{13}C$-NMR gesichert. Durch

einen $^{13}C$-NMR-spektroskopischen Vergleich mit dem 4,4'-Diisocyanto-diphenylsulfid konnte eindeutig nachgewiesen werden, daß die p-ständige Isocyanatgruppe im erfindungsgemäßen Diisocyanat in dimerisierter Form vorliegt.

Beispiele 2 - 5

In den nachfolgenden Beispielen 2 - 5 wurden folgende Diamine eingesetzt (die Herstellung erfolgte in Analogie zu DE-OS 2 948 419):

Diamin I

A) 13,85 kg (3,46 Mol) eines linearen Polyethers der OH-Zahl 28, der durch Umsetzung von Propylenglykol mit einem Gemisch aus 79 % Propylenoxid und 21 % Ethylenoxid erhalten wurde, werden nach sorgfältigem Entwässern zu 1,205 kg (6,92 Mol) 2,4-Toluylendiisocyanat bei 80°C unter Rühren zugefügt und das Rühren bei 80°C 2 Stunden fortgesetzt. Der NCO-Gehalt des Präpolymeren beträgt 1,9 % (berechnet: 1,93 %).

B) Eine Lösung von 15,1 kg des nach A hergestellten Präpolymeren (6,83 Mol NCO) in 15 l Dioxan wird bei 15 - 25°C langsam zu einer Lösung von 332 g NaOH (8,3 Mol) und 15 g eines handelsüblichen Emulgators (K-Salz einer technischen Alkylbenzolsulfonsäure) in 15 l Wasser gegeben und 30 Minuten bei

dieser Temperatur weitergerührt. Danach werden 1205 ml konzentrierte HCl (14,5 Mol) zugegeben, wobei stürmische Gasentwicklung einsetzt. Nachdem mit 247 g NaOH (6,2 Mol) neutralisiert worden ist, wird die organische Phase abgetrennt und das Lösungsmittel im Vakuum abdestilliert. Das resultierende Rohdiamin wird in Methylenchlorid gelöst und mit Wasser gewaschen. Nach dem Trocknen bei 50°C/15 Torr und dann 50°C/0,01-0,2 Torr hinterbleiben 14,5 kg eines Öles mit der Viskosität 15 000 mPa.s bei 26°C.

Molekulargewicht: 4700 (dampfdruckosmometrisch)
NH-Zahl: 20,9

Diamin II

A) 912 g (2,155 Mol) eines sorgfältig entwässerten linearen Polyethers der OH-Zahl 265, der durch Propoxylierung von Propylenglykol erhalten wurde, werden zu 750 g (4,39 Mol) 2,4-Toluylendiisocyanat bei 80°C unter Rühren zugefügt und das Rühren bei 80°C 1 Stunde fortgesetzt. Der NCO-Gehalt des Semi-Präpolymeren beträgt 10,85 % (berechnet: 10,9 %).

B) Eine Lösung von 1726 g (4,46 Mol NCO) des nach A hergestellten Präpolymeren in 800 ml Dioxan wird bei 22 - 25°C langsam zu einer Lösung von 260 g NaOH (6,5 Mol) und 3 g eines handelsüblichen Emulgators (K-Salz einer technischen Alkylbenzolsulfonsäure)

in 1 l Wasser gegeben und 1 Stunde bei dieser Temperatur weitergerührt. Danach werden 708 ml konz. HCl (8,5 Mol) zugegeben, wobei stürmische Gasentwicklung einsetzt. Nachdem mit 80 g NaOH (2,0 Mol) neutralisiert worden ist wird die organische Phase abgetrennt und das Lösungsmittel im Vakuum abdestilliert. Aufarbeitung wie Diamin I.

| | |
|---|---|
| Ausbeute: | 1300 g |
| Viskosität: | 7440 mPa.s bei 75°C |
| Molekulargewicht: | 950 (dampfdruckosmometrisch) |
| NH-Zahl: | 128 |

Wie nachstehend in Tabelle 1 mitgeteilt, werden die obengenannten Diamine mit den in der Tabelle angegebenen Mengen des erfindungsgemäßen, Uretdiongruppen aufweisenden Diisocyanats in Toluol umgesetzt. Die Isolierung der dabei erhaltenen Umsetzungsprodukte erfolgte durch deren Filtration nach Beendigung der Umsetzung. Die Beendigung der Umsetzung wurde am Verschwinden der NCO-Bande im IR-Spektrum des Reaktionsgemischs festgestellt.

Tabelle 1

| Nr. | Amin I | | Amin II | | Uretdiondiisocyanat | | Lösungsmittel und Menge | Reaktions-temp. | Ausbeute | Schmelzp. | Verhältnis fr.$NCO/NH_2$ |
|---|---|---|---|---|---|---|---|---|---|---|---|
| | g | Äquiv.$NH_2$ .1000 | g | Äquiv.$NH_2$ .1000 | g | Äquiv. freies NCO .1000 | | | | | |
| 2 | 20 | 8,5 | 80 | 168 | 23,6 | 88 | Toluol, 800ml | 50°C | 89 % | 131-141°C | 1:2,0 |
| 3 | 40 | 17 | 60 | 126 | 19 | 70 | Toluol, 800ml | 50°C | 81 % | 131-136°C | 1:2,0 |
| 4 | 20 | 8,5 | 30 | 63 | 9,5 | 35,4 | Toluol, 550ml | 100°C | 82,3 % | 133-140°C | 1:2,0 |
| 5 | 15 | 6,4 | 22,5 | 47,4 | 9,5 | 35,4 | Toluol, 470ml | 100°C | 96 % | 140-145°C | 1:1,52 |

Beispiele 6 - 13

In den nachfolgenden Beispielen 6 - 13 (vgl. Tabelle 2) wurden Proben der festen Umsetzungsprodukte aus den Beispielen 2 - 5 auf einer Stahlplatte auf die in Tabelle 2 angegebenen Temperaturen erhitzt. Nach dem Abkühlen wurde die Oberflächenhärte der ca. 2 - 3 mm dicken Probekörper gemessen.

Tabelle 2

| Beispiel | Ausgangsprodukt Beispiel Nr. | Dauer der Erwärmung | Temperatur | Shore A Oberflächenhärte DIN 53 505 |
|---|---|---|---|---|
| 6 | 2 | 30 Min. | 150°C | 80 |
| 7 | 2 | 30 Min. | 170°C | 85 |
| 8 | 3 | 30 Min. | 150°C | 85 |
| 9 | 3 | 30 Min. | 170°C | 90 |
| 10 | 4 | 30 Min. | 150°C | 60 |
| 11 | 4 | 30 Min. | 170°C | 65 |
| 12 | 5 | 30 Min. | 150°C | 70 |
| 13 | 5 | 30 Min. | 170°C | 75 |

Patentansprüche

1. Uretdiongruppen aufweisendes Diisocyanat der Formel

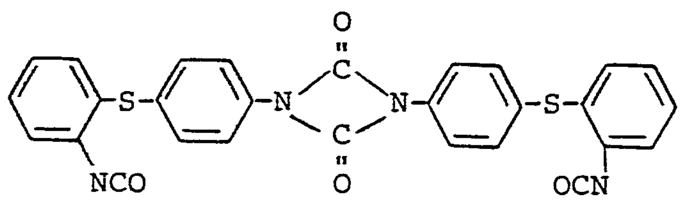

2. Verfahren zur Herstellung von hitzevernetzbaren, aromatisch gebundene primäre Aminogruppen und Uretdiongruppen aufweisenden Oligoadditionsprodukten, dadurch gekennzeichnet, daß man das Uretdiongruppen aufweisende Diisocyanat der Formel

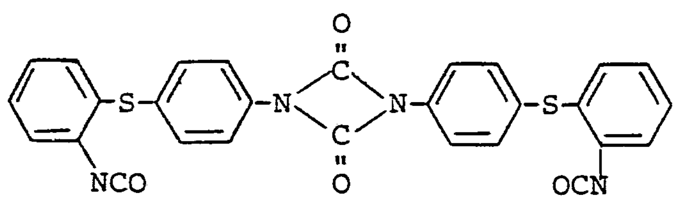

mit mindestens zwei aromatisch gebundene, primäre Aminogruppen aufweisenden organischen Verbindungen des Molekulargewichtsbereichs 500 bis 10 000 unter Einhaltung eines $NCO/NH_2$-Molverhältnisses von 1:1,05 bis 1:2 umsetzt.

3. Verfahren gemäß Anspruch 2, dadurch gekennzeichnet, daß man die Umsetzung unter Einhaltung eines $NCO/NH_2$-Molverhältnisses von 1:1,4 bis 1:2 durchführt.

4. Verfahren gemäß Anspruch 2 und 3, dadurch gekennzeichnet, daß man die Umsetzung im Temperaturbereich von 30 bis 120°C durchführt.

5. Verfahren gemäß Anspruch 2 bis 4, dadurch gekennzeichnet, daß man die Umsetzung in Gegenwart von inerten Lösungsmitteln durchführt.

6. Verwendung der gemäß Anspruch 2 bis 5 erhältlichen, aromatisch gebundene primäre Aminogruppen und Uretdiongruppen aufweisenden Oligoadditionsprodukte zur Herstellung von Harnstoffgruppen aufweisenden, hochmolekularen Kunststoffen, dadurch gekennzeichnet, daß man die Oligoadditionsprodukte auf eine Temperatur von oberhalb 130°C erhitzt.

Europäisches Patentamt

# EUROPÄISCHER RECHERCHENBERICHT

0074023
Nummer der Anmeldung

EP 82 10 7829

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| | --- | | C 07 D 229/00<br>C 08 G 18/00 |
| A | DE-A-2 524 476 (BAYER) | | |
| | ----- | | |
| | | | RECHERCHIERTE SACHGEBIETE (Int. Cl. 3) |
| | | | C 07 D 229/00 |

Der vorliegende Recherchenbericht wurde für alle Patentansprüche erstellt.

| Recherchenort | Abschlußdatum der Recherche | Prüfer |
|---|---|---|
| DEN HAAG | 13-12-1982 | DE BUYSER I.A.F. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument